Europäisches Patentamt

European Patent Office

Office européen des brevets

(11) Numéro de publication: **0 311 190**
**A1**

# (12) DEMANDE DE BREVET EUROPEEN

(21) Numéro de dépôt: 88202132.2

(51) Int. Cl.⁴: **A61B 6/04**

(22) Date de dépôt: 29.09.88

(30) Priorité: 06.10.87 FR 8713771

(43) Date de publication de la demande:
12.04.89 Bulletin 89/15

(84) Etats contractants désignés:
BE DE FR GB IT NL

(71) Demandeur: PHILIPS SYSTEMES MEDICAUX
177, rue de Bezons
F-78420 Carrières sur Seine(FR)

(84) FR

(71) Demandeur: N.V. Philips'
Gloeilampenfabrieken
Groenewoudseweg 1
NL-5621 BA Eindhoven(NL)

(84) BE DE GB IT NL

(72) Inventeur: Louiday, André Emile
Société Civile S.P.I.D. 209, rue de l'Université
F-75007 Paris(FR)

(74) Mandataire: Landousy, Christian et al
Société Civile S.P.I.D. 209, Rue de
l'Université
F-75007 Paris(FR)

(54) Dispositif radiologique de type basculant.

(57) Dispositif radiologique de type basculant comprenant un socle (1) fixe, un chariot (2) basculant longitudinal assujetti au socle (1) et portant d'une part longitudinalement un panneau porte-patient (8) lié au chariot par un support de panneau porte-patient, support de panneau constitué d'une potence (3) dont la poutre (5) haute horizontale assujettie à un bras (6) forme un pont pour le passage d'un praticien.

EP 0 311 190 A1

## DISPOSITIF RADIOLOGIQUE DE TYPE BASCULANT

L'invention concerne un dispositif radiologique de type basculant comprenant un socle, un chariot basculant longitudinal assujetti au socle et portant d'une part longitudinalement, un panneau porte-patient lié au chariot par un support de panneau porte-patient et d'autre part un support d'arceau muni d'un arceau qui porte lui-même un système d'analyse X, composé d'une source de rayonnement X et d'un récepteur de rayonnement X.

Un exemple d'un dispositif radiologique est donné dans le brevet 2 565 093. Ce brevet décrit un dispositif radiologique comprenant un chariot et un panneau porte-patient lié audit chariot par l'intermédiaire d'un support sensiblement horizontal de manière à aménager entre le panneau porte-patient et le chariot un espace suffisant pour le passage d'un praticien.

Actuellement, les dispositifs de radiodiagnostic sont utilisés de préférence en chirurgie et permettent alternativement d'une part, une analyse radiologique d'une partie déterminée du corps humain et d'autre part, le traitement chirurgical de cette partie, et cela, en fonction des besoins des praticiens.

Le dispositif radiologique décrit dans le brevet cité ne permet pas une telle alternance et possède des inconvénients comme par exemple,

- L'espace existant entre le chariot et la table porte-patient est fixe.

- Le chariot doit avoir une longueur importante pour pouvoir dégager un espace entre d'une part, l'arceau d'analyse X déplacé alors à l'extrémité du chariot et d'autre part, la partie extrême du panneau porte-patient.

- Dans cette position le basculement du panneau porte-patient n'est plus possible, l'arceau d'analyse X venant alors en contact avec le sol.

- Lorsque le praticien désire utiliser le système d'analyse X pendant une opération d'exploration ou le traitement chirurgical, l'accès total au patient n'est plus possible.

L'invention a pour objet un dispositif radiologique pouvant être utilisé simultanément en radiodiagnostic et en chirurgie du corps humain, le contrôle ou l'exploration par analyse X pouvant se faire en ayant accès de part et d'autre du patient allongé sur le panneau porte-patient, sans gène ni encombrement.

Elle se caractérise par le fait que le support de panneau est constitué d'une potence, solidaire du chariot basculant, fixée à une extrémité dudit chariot et dont la poutre haute horizontale porte à son extrémité libre, un bras perpendiculaire à la poutre, bras muni d'une équerre portant le panneau porte-patient par une de ses extrémités, la potence et le bras formant un pont pour le passage d'un praticien.

La potence placée à une extrémité du chariot permet de compenser une partie de la charge du système d'analyse X, l'équilibrage des masses de part et d'autre des moyens de rotation du chariot permet de réduire la puissance du moteur dont la fonction est l'entrainement à rotation dudit chariot.

Le pont formé par le support de panneau porte-patient offre un passage pour l'accès des praticiens de part et d'autre du patient pendant l'utilisation du système d'analyse X.

Le chariot basculant est de longueur sensiblement égale à la longueur du panneau porte-patient.

Le passage entre le panneau porte-patient et le chariot permet, par une rotation cylindrique de l'arceau autour de son axe central et une rotation du support d'arceau autour de son axe de rotation d'inverser symétriquement la po sition de la source de rayonnement X et dutube récepteur de rayonnement X par rapport au panneau porte-patient.

Dans une forme particulière de l'invention la poutre est une coulisse dans laquelle glisse un coulisseau horizontal solidaire du bras, ce qui permet de faire varier l'espace existant entre le chariot basculant et le panneau pour :

- positionner le patient par rapport au système d'analyse X,

- éloigner le patient du système d'analyse,

- ouvrir un espace important autour du patient.

Préférentiellement le bras est muni d'une glissière assujettie à l'équerre pour un déplacement du panneau porte-patient dans des plans perpendiculaires à l'axe longitudinal du bras.

La glissière par son mouvement permet de placer le patient correctement par rapport au système d'analyse X et de régler la hauteur du panneau par rapport au sol de tel sorte que les praticiens puissent opérer dans de bonnes conditions.

La description ci-après, en se référant aux dessins annexés, le tout donné à titre d'exemple non limitatif fera bien comprendre comment l'invention peut être réalisée.

La figure 1 est une vue de profil du dispositif radiologique selon l'invention.

La figure 2 est une vue de face du dispositif de la figure 1.

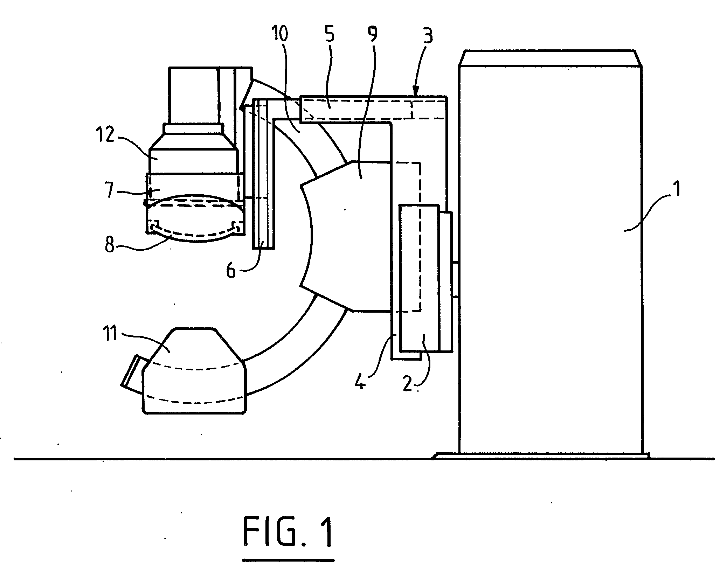

La figure 1 présente un dispositif radiologique de type basculant comprenant un socle 1 sensiblement parallélépipédique devant lequel est placé un chariot 2 basculant et pouvant être déplacé verticalement par des moyens mécaniques connus. Le chariot 2 porte à une de ses extrémités perpendiculairement à son axe longitudinal une potence 3 formée d'un mât 4 et d'une poutre 5 horizontale. A l'extrémité libre de la poutre 5 de la potence 3, est aménagé un bras 6 placé, dans le plan formé par le mât 4 et la poutre 5, perpendiculairement à ladite poutre, les trois éléments 4, 5, 6 formant un pont sous lequel peut passer un praticien. L'extrémité du bras 6 est muni d'une équerre 7 permettant le montage d'un panneau porte-patient 8, le panneau porte-patient étant sur la figure 1 vu de bout.

Il est représenté sur la figure 1 l'image d'un support d'arceau 9 monté à rotation et translation sur le chariot 2 basculant. Le support 9 est muni d'un arceau 10 portant lui-même un système d'analyse X composé d'une source 11 de rayonnement X en vis-à-vis duquel est placé un tube 12 récepteur de rayonnement. La source 11 et le tube 12 sont placés de part et d'autre du patient lorsque celui-ci est allongé sur le panneau porte-patient 8, lors d'une analyse radiologique.

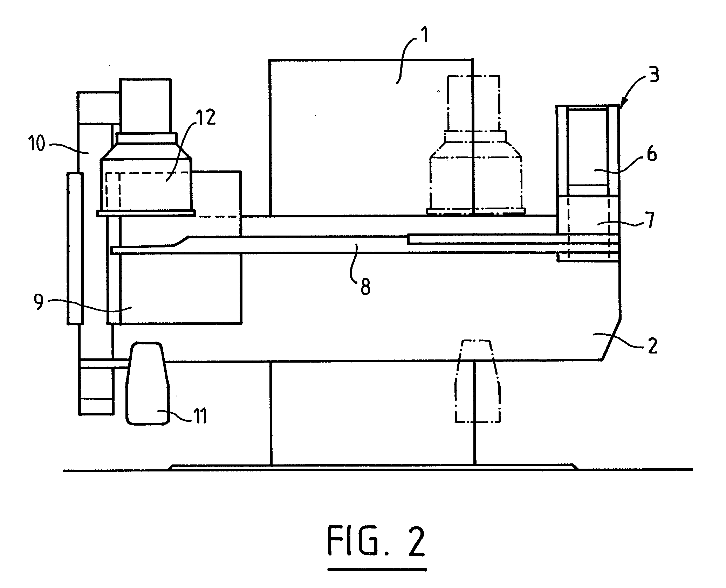

La figure 2 montre le dispositif radiologique vu de face. Devant le socle 1 est placé le chariot 2 basculant à une extrémité duquel est aménagée la potence 3, avec le bras 6, l'équerre 7 porte panneau et le panneau porte-patient 8. L'arceau 10 et son support 9 munis du système d'analyse X placé sur la figure à l'autre extrémité du chariot peut se déplacer longitudinalement au chariot, la source 11 et le tube 12 étant de part et d'autre du panneau porte-patient. L'image en traits mixtes fins donne une autre position de l'arceau lors d'un déplacement horizontal de celui-ci.

Par rotation cylindrique de l'arceau et par pivotement autour de l'axe de rotation du support d'arceau, il est possible d'inverser la position de la source et du tube récepteur, la source X se déplaçant alors au-dessus du panneau, le tube analyseur étant en-dessous. L'inversion est facilitée par le fait que le panneau porte-patient est monté en porte à faux et éloigné du chariot, l'inversion étant réalisée par des mouvements de rotation de l'arceau de part et d'autre du panneau porte-patient sans qu'il soit nécessaire de déplacer ledit panneau.

## Revendications

1. Dispositif radiologique de type basculant comprenant un socle, un chariot basculant longitudinal assujetti au socle et portant d'une part longitudinalement un panneau porte-patient lié au chariot par un support de panneau porte-patient et d'autre part un support d'arceau muni d'un arceau qui porte lui-même un système d'analyse fixe composé, d'une source de rayonnement X et d'un récepteur de rayonnement X, caractérisé en ce que le support de panneau est constitué d'une potence solidaire du chariot basculant, fixée à une extrémité dudit chariot et dont la poutre haute horizontale porte à son extrémité libre, un bras perpendiculaire à la poutre, bras muni d'une équerre portant le panneau porte-patient par une de ses extrémités, la potence et le bras formant un pont pour le passage d'un praticien.

2. Dispositif radiologique de type basculant selon la revendication 1, caractérisé en ce que la poutre est une coulisse dans laquelle glisse un coulisseau horizontal solidaire du bras.

3. Dispositif radiologique de type basculant selon la revendication 1 et 2, caractérisé en ce que le bras est muni d'une glissière assujettie à l'équerre pour un déplacement du panneau porte-patient dans des plans perpendiculaires à l'axe longitudinal du bras.

FIG. 1

EP 0 311 190 A1

EP 0 311 190 A1

FIG. 2

2-II-PHF 87-577

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 165 157 (THOMSON-CGR)<br>* Page 2, lignes 1-23, ligne 29 - page 3, ligne 25; page 4, ligne 15 - page 5, ligne 8, ligne 33 - page 6, ligne 15; figure * & FR-A-2 565 093 (Cat. A)<br>--- | 1 | A 61 B 6/04 |
| Y | US-A-4 653 083 (R.J. ROSSI)<br>* Résumé; colonne 1, lignes 20-33; colonne 2, lignes 1-17,39-50; figures 1,2 *<br>--- | 1,3 | |
| Y | FR-A-1 155 980 (ANCIENS ETS. ALEXANDRE)<br>* Page 1, colonne de droite, lignes 4-21; page 2, colonne de gauche, lignes 12-18,28-40; figures 1,2 * | 1,3 | |
| A | | 2 | |
| A | ---<br>DE-A-2 046 207 (J.W. PEGRUM et al.)<br>* Page 4, lignes 13-18; page 6, lignes 7-21; page 8, lignes 4-13; page 10, lignes 18-23; figures 1,2 *<br>----- | 1-3 | |

DOMAINES TECHNIQUES
RECHERCHES (Int. Cl.4)

A 61 B

Le présent rapport a été établi pour toutes les revendications

| Lieu de la recherche | Date d'achèvement de la recherche | Examinateur |
|---|---|---|
| LA HAYE | 25-11-1988 | RIEB K.D. |

EPO FORM 1503 03.82 (P0402)